# FASCICULE DE BREVET EUROPEEN

(11) **EP 3 773 378 B1**
(45) Date de publication et mention de la délivrance du brevet: **15.03.2023**
(21) Numéro de dépôt: 19714450.4
(22) Date de dépôt: 05.04.2019
(51) Int. Cl.: A61F 9/008

(54) **APPAREIL DE TRAITEMENT D'UN TISSU INCLUANT DES SYSTEMES OPTIQUES ORIGINAUX DE DEVIATION ET DE FOCALISATION D'UN FAISCEAU L.A.S.E.R**
GERÄT ZUR BEHANDLUNG EINES GEWEBES, EINSCHLIESSLICH ORIGINALER OPTISCHER SYSTEME ZUR ABLENKUNG UND FOKUSSIERUNG EINES LASERSTRAHLS
APPLIANCE FOR TREATING A TISSUE, INCLUDING ORIGINAL OPTICAL SYSTEMS OF DEFLECTION AND FOCUSING OF A LASER BEAM

(30) Priorité: 06.04.2018 FR 1870407
(43) Date de publication de la demande: 17.02.2021
(73) Titulaire: KERANOVA, 42000 Saint Etienne (FR)
(72) Inventeur: BAUBEAU, Emmanuel, 42000 Saint Etienne (FR)
(74) Mandataire: Be IP
(86) Numéro de dépôt international: PCT/EP2019/058627
(87) Numéro de publication internationale: WO 2019/193148

(56) Documents cités:
- WO-A1-2015/178803
- WO-A1-2017/174710

## Description

### DOMAINE TECHNIQUE

La présente invention concerne le domaine technique des opérations chirurgicales réalisées au laser femtoseconde, et plus particulièrement celui de la chirurgie ophtalmologique pour notamment des applications de découpes de cornées, ou de cristallins.

L'invention concerne un dispositif de découpe d'un tissu humain ou animal, telle qu'une cornée, ou un cristallin, au moyen d'un laser femtoseconde.

Par laser femtoseconde, on entend une source lumineuse, apte à émettre un faisceau L.A.S.E.R. sous forme d'impulsions ultra-courtes, dont la durée est comprise entre 1 femtoseconde et 100 picosecondes, de préférence comprise entre 1 et 1000 femtosecondes, notamment de l'ordre de la centaine de femtosecondes.

### ART ANTERIEUR

Les lasers femtoseconde sont couramment utilisés en chirurgie pour la découpe de la cornée ou du cristallin. Ils délivrent des impulsions ultra-brèves et de forte puissance, voir par exemple WO 2015 178 803 ou WO 2017 174 710.

Lors d'une chirurgie de la cornée, le laser femtoseconde peut être utilisé pour réaliser une découpe du tissu cornéen, en focalisant un faisceau L.A.S.E.R. dans le stroma de la cornée. Plus précisément, à chaque impulsion, le laser femtoseconde génère un faisceau. Ce faisceau est focalisé (en un point dit « de focalisation » situé) dans la cornée. Une bulle de gaz se forme au point de focalisation, engendrant une disruption très localisée des tissus environnant. Une partie de l'énergie du faisceau est consommée lors de la génération de la bulle de gaz. Le reste de l'énergie du faisceau se propage jusqu'à la rétine, ce qui provoque un échauffement local de la rétine.

Pour former une ligne de découpe dans la cornée, une succession de petites bulles de gaz adjacentes sont générées en déplaçant le faisceau. Pour déplacer le faisceau, un scanner de balayage 1 est utilisé. Ce scanner de balayage 1 est généralement composé de miroirs galvanométriques pilotables et/ou de platines permettant le déplacement d'éléments optiques, tels que des miroirs.

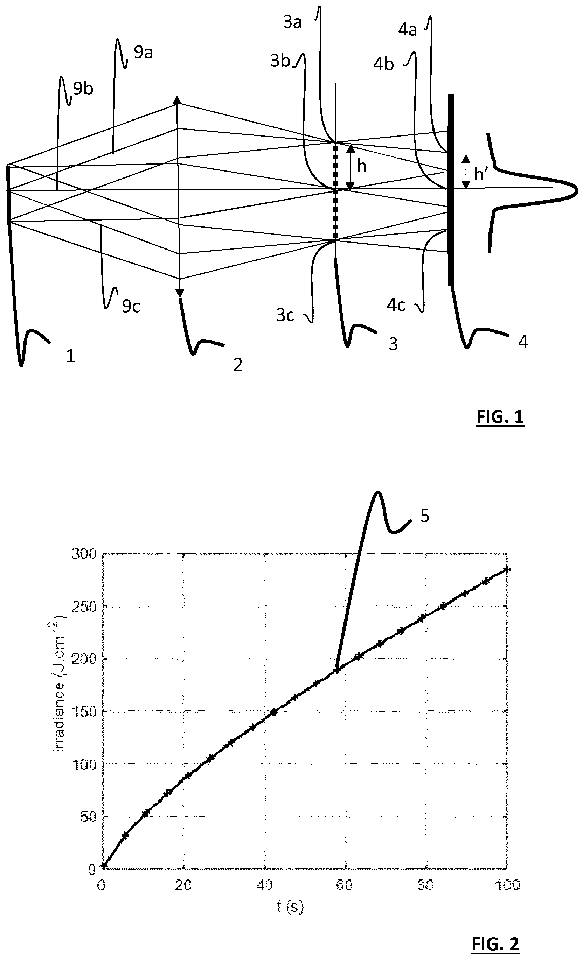

En référence à la figure 1, à chaque impulsion, le faisceau issu du laser femtoseconde :
- pénètre dans le scanner de balayage 1,
- traverse un ensemble de focalisation 2,
- focalise en un point de focalisation dans la cornée 3 pour former une bulle de gaz, puis
- diverge vers la rétine 4 du patient.

Cette divergence du faisceau tend à échauffer la rétine 4.

Pour réduire les risques de dégradation de la rétine 4 lors d'une exposition à un faisceau généré par un laser femtoseconde, des éclairements énergétiques (ou *« irradiances »)* limites ont été calculées pour l'œil d'un patient en fonction du temps. Le document intitulé « ICNIRP Guidelines on limits of exposure to LASER radiation of wavelengths between 180 nm AND 1,000 mm » de « International Commission on Non-Ionizing Radiation Protection » décrit de tels éclairements énergétiques limites en fonction de la (ou des) longueur(s) d'onde(s) d'émission du laser et de la durée d'exposition au faisceau généré par le laser femtoseconde (voir notamment les tableaux 5 et 6 de ce document).

A titre indicatif, une courbe 5 illustrant les expositions énergétiques limites (exprimés en Joules/cm²) en fonction d'un temps est représentée à la figure 2.

Lors de l'utilisation d'un laser femtoseconde dans le traitement d'une pathologie oculaire, il est nécessaire que l'exposition énergétique de la rétine (pour un temps d'exposition de la rétine donné) soit inférieur à une exposition énergétique limite définit par la courbe illustrée à la figure 2. Ceci permet de garantir l'intégrité de la rétine lors de l'utilisation du laser femtoseconde dans le traitement de la pathologie oculaire.

Pour découper une cornée sur une surface de 1mm², il faut réaliser environ 20000 impacts très proches les uns des autres. Ces impacts sont réalisés un par un à une vitesse moyenne de 300000 impacts/seconde. Pour découper une cornée sur une surface d'environ 65mm², en tenant compte des temps pendant lesquels le laser arrête la production des impulsions en bout de segment pour permettre aux miroirs de se positionner sur le segment suivant, il faut en moyenne 15 secondes. L'opération chirurgicale de découpe est donc lente.

Pour optimiser le temps de découpe, il est connu d'augmenter la fréquence du laser. Cependant, l'augmentation de la fréquence implique également une augmentation de la vitesse de déplacement du faisceau, au moyen de platines ou de scanners adaptés. Il est également connu d'augmenter l'espacement entre les impacts du laser sur le tissu à découper, mais généralement au détriment de la qualité de la découpe.

Une autre solution pour diminuer le temps de découpe consiste à générer plusieurs bulles de gaz simultanément. Le fait de générer simultanément « *n* » bulles de gaz permet de diminuer la durée totale de la découpe d'un facteur supérieur à « *n* » (car on réduit également le nombre d'aller-retour que doit effectuer le faisceau pour traiter toute une surface du tissu).

La génération simultanée d'une pluralité de bulles de gaz nécessite d'augmenter la puissance du laser femtoseconde afin qu'en chaque point de focalisation, la puissance issue du laser femtoseconde soit suffisante pour générer une bulle de gaz respective.

Cette augmentation de puissance induit une augmentation de l'exposition énergétique reçu par la rétine, qui peut alors dépasser les expositions énergétiques limites donnés par le document « ICNIRP Guidelines on limits of exposure to LASER radiation of wavelengths between 180 nm AND 1,000 mm » de « International Commission on Non-Ionizing Radiation Protection »*.*

Un but de la présente invention est de proposer un dispositif de traitement d'une pathologie oculaire incluant un laser femtoseconde de forte puissance et qui permet de respecter les contraintes liées aux expositions énergétiques limites que peut supporter une rétine.

### EXPOSE DE L'INVENTION

A cet effet l'invention propose un appareil de traitement d'un tissu humain ou animal, tel qu'une cornée ou un cristallin, ledit appareil incluant un dispositif de conditionnement d'un faisceau L.A.S.E.R. généré par un laser femtoseconde, le dispositif de conditionnement étant disposé en aval du laser femtoseconde et comprenant un scanner optique de balayage et un système optique de focalisation en aval du scanner optique de balayage :
- le scanner optique de balayage comportant au moins un miroir optique pivotant autour d'au moins un axe pour dévier le faisceau L.A.S.E.R.,
- le système optique de focalisation comportant un module concentrateur pour focaliser le faisceau L.A.S.E.R. dans un plan de focalisation,
   ***remarquable en ce que*** ledit au moins un miroir optique pivotant du scanner optique de balayage est positionné sur le chemin optique du faisceau L.A.S.E.R. entre :
   ∘ le plan focal objet F_{Objet} d'une lentille équivalente au système optique de focalisation, et
   ∘ un orifice d'entrée du système optique de focalisation.

Des aspects préférés mais non limitatifs de l'appareil son les suivants :
une pupille d'entrée du module concentrateur est positionnée dans le plan de la lentille équivalente au système optique de focalisation ;
- le système optique de focalisation comporte un dispositif de relais optique en amont du module concentrateur ;
- le dispositif de relais optique est positionné le long du chemin optique du faisceau L.A.S.E.R. de sorte à former une image d'une zone au voisinage dudit au moins un miroir optique pivotant dans le plan de la lentille équivalente au système optique de focalisation ;
- le dispositif de conditionnement comprend en outre un système de mise en forme du faisceau, pour moduler la phase du front d'onde du faisceau L.A.S.E.R. de sorte à obtenir un faisceau L.A.S.E.R. modulé en phase selon une consigne de modulation calculée pour répartir l'énergie du faisceau L.A.S.E.R. en au moins deux points d'impact formant un motif dans le plan de focalisation ;
- le dispositif de conditionnement comprend en outre une unité de commande pour piloter le laser femtoseconde, le scanner optique de balayage et le système optique de focalisation ;
- le module concentrateur peut être déplaçable en translation le long du chemin optique du faisceau L.A.S.E.R. entre des première et deuxième positions extrêmes, le module concentrateur étant plus proche du dispositif de relais optique dans la première position extrême que dans la deuxième position extrême ;
- l'appareil de découpe peut comprendre en outre un dispositif optique pré-compensatoire positionné en amont du système optique de focalisation de sorte à générer une aberration compensatoire en sortie du système optique de focalisation, ladite aberration compensatoire permettant de compenser les aberrations produites sur le faisceau L.A.S.E.R. notamment par la traversée du tissu humain ou animal, l'unité de commande étant adaptée pour commander le déplacement en translation du module concentrateur en fonction de la distance estimée par le calculateur.

### BREVE DESCRIPTION DES DESSINS

D'autres caractéristiques et avantages de l'invention ressortiront clairement de la description qui en est réalisée ci-après, à titre indicatif et nullement limitatif, en référence aux figures annexées, dans lesquelles :
- la figure 1 est une représentation schématique d'un exemple de dispositif de traitement d'une pathologie oculaire utilisant un laser femtoseconde ;
- la figure 2 est une représentation schématique d'une courbe d'irradiance limite en fonction du temps,
- la figure 3 est une représentation schématique d'un montage incluant l'appareil de découpe selon l'invention ;

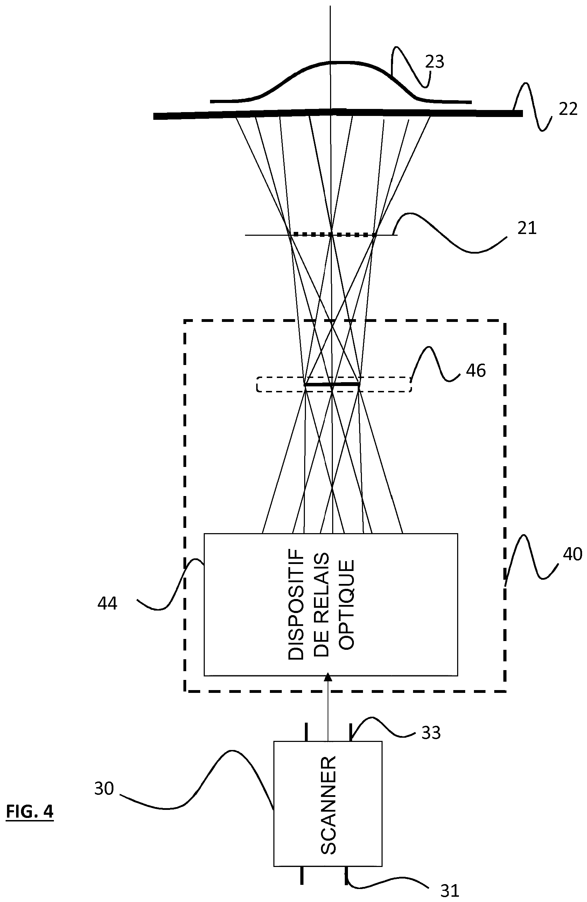

- les figures 4 à 6 sont des représentations schématiques d'un scanner optique de balayage et d'un système optique de focalisation de l'appareil de découpe selon l'invention.

### EXPOSE DETAILLE DE L'INVENTION

L'invention concerne un appareil de traitement d'une pathologie oculaire. Plus précisément, l'invention concerne un appareil de découpe d'un tissu humain au moyen d'un laser femtoseconde. Dans la suite de la description, l'invention sera décrite, à titre d'exemple, pour la découpe d'une cornée d'un oeil humain ou animal.

### 1. Définitions

On entend, dans le cadre de la présente invention, par *« point d'impact »* une zone du faisceau L.A.S.E.R. comprise dans son plan focal dans laquelle l'intensité dudit faisceau L.A.S.E.R. est suffisante pour générer une bulle de gaz dans un tissu.

On entend, dans le cadre de la présente invention, par *« points d'impact adjacents »,* deux points d'impact disposés en regard l'un de l'autre et non séparés par un autre point d'impact. On entend par « *points d'impact voisins »* deux points d'un groupe de points adjacents entre lesquels la distance est minimale.

On entend, dans le cadre de la présente invention, par *« motif »* une pluralité de points d'impact L.A.S.E.R. générés simultanément dans un plan de focalisation d'un faisceau L.A.S.E.R. mis en forme - c'est-à-dire modulé en phase pour répartir son énergie en plusieurs spots distincts dans le plan de focalisation correspondant au plan de découpe du dispositif.

### 2. Appareil de découpe

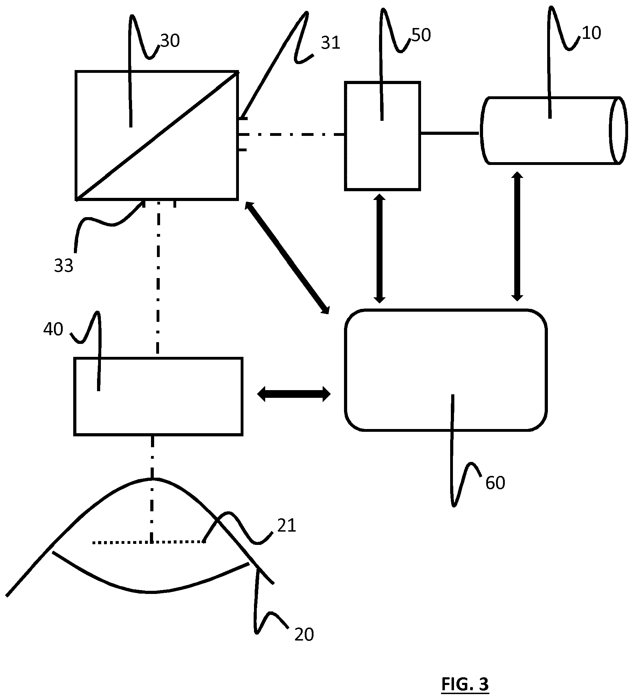

En référence à la figure 3, on a illustré un mode de réalisation de l'appareil de découpe selon l'invention. Celui-ci peut être disposé entre un laser femtoseconde 10 et une cible à traiter 20.

Le laser femtoseconde 10 est apte à émettre un faisceau L.A.S.E.R. sous la forme d'impulsions. A titre d'exemple, le laser 10 émet une lumière de 1030 nm de longueur d'onde, sous la forme d'impulsions de 400 femtosecondes. Le laser 10 possède une puissance de 20W et une fréquence de 500 kHz.

La cible 20 est par exemple un tissu humain ou animal à découper tel qu'une cornée ou un cristallin.

L'appareil de découpe comprend un dispositif de conditionnement de faisceau incluant :
- un scanner optique de balayage 30 en aval du laser 10,
- un système optique de focalisation 40 en aval du scanner optique de balayage 30.

Le dispositif de conditionnement inclut également une unité de commande 60 permettant de piloter le scanner optique de balayage 30 et le système optique de focalisation 40.

Le scanner optique de balayage 30 permet d'orienter le faisceau issu du laser 10 pour le déplacer le long d'un chemin de déplacement prédéfini par l'utilisateur dans un plan de focalisation 21.

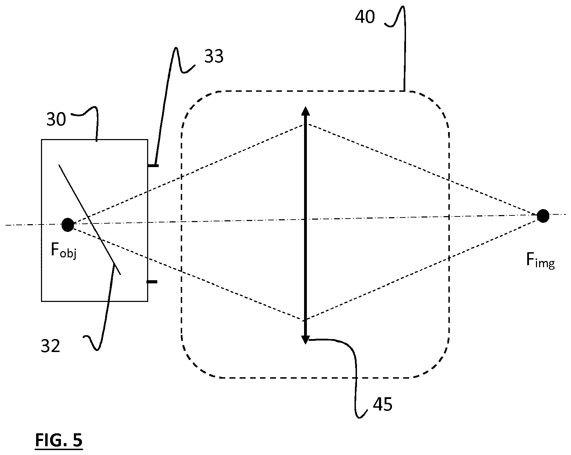

Le système optique de focalisation 40 permet de focaliser le faisceau dans le plan de focalisation 21 - correspondant au plan de découpe. A titre indicatif, on a illustré à la figure 5 une lentille équivalente 45 au système optique de focalisation 40 dans son ensemble, étant bien entendu pour l'homme du métier que le système optique de focalisation ne consiste pas uniquement en une lentille fixe.

Le dispositif de conditionnement peut également inclure un système de mise en forme 50 entre le laser femtoseconde 10 et le scanner optique de balayage 30. Ce système de mise en forme 50 est positionné sur la trajectoire du faisceau issu du laser femtoseconde 10. Le système de mise en forme 50 permet de moduler la phase du faisceau issu du laser femtoseconde 10 pour répartir l'énergie du faisceau en une pluralité de points d'impact dans son plan focal, cette pluralité de points d'impact définissant un motif.

Ainsi, le système de mise en forme 50 permet de générer simultanément plusieurs points d'impact définissant un motif, le scanner optique de balayage 30 permet de déplacer ce motif dans le plan de focalisation 21, et le système optique de focalisation 40 permet de focaliser le faisceau dans le plan de focalisation 21.

Les différents éléments constituant le dispositif de conditionnement vont maintenant être décrits plus en détails en référence aux figures.

### 3. Eléments du dispositif de conditionnement

### 3.1. Scanner optique de balayage

Le scanner optique de balayage 30 permet de dévier le faisceau (éventuellement modulé en phase) de sorte à le déplacer en une pluralité de positions dans le plan de focalisation 21 correspondant au plan de découpe.

Le scanner optique de balayage 30 comprend :
- un orifice d'entrée 31 pour recevoir le faisceau (éventuellement modulé en phase par l'unité de mise en forme 50),
- un (ou plusieurs) miroir(s) optique(s) 32 pivotant autour d'un (ou plusieurs) axe(s) pour dévier le faisceau L.A.S.E.R., et
- un orifice de sortie 33 pour envoyer le faisceau L.A.S.E.R. vers le système optique de focalisation 40.

Le scanner optique 30 utilisé est par exemple une tête de balayage IntelliScan III de la société SCANLAB AG. Le (ou les) miroir(s) 32 du scanner optique est (sont) connecté(s) à un (ou des) moteur(s) pour permettre leur pivotement. Ce(s) moteur(s) pour le pivotement du (ou des) miroir(s) 32 est (sont) avantageusement piloté(s) par l'unité de l'unité de commande 60 qui sera décrite plus en détails dans la suite.

Avantageusement, et comme illustré à la figure 5, le (ou les) miroir(s) optique(s) 32 pivotant du scanner optique de balayage 30 est (sont) positionné(s) entre un plan focal objet F_{Objet} du système optique de focalisation 40 et le système de focalisation 40. Ceci permet d'augmenter l'angle de balayage en sortie du système de focalisation 40 de sorte que l'énergie émise par le laser femtoseconde soit répartie sur une plus grande surface de la rétine.

Il est ainsi possible d'augmenter la puissance du laser femtoseconde 10 tout en maintenant l'éclairement énergétique reçu par la rétine dans une gamme de valeur inférieure aux éclairements énergétiques limites donnés par le document « *ICNIRP Guidelines on limits of exposure to LASER radiation of wavelengths between 180 nm AND 1,000 mm »* de *« International Commission on Non-Ionizing Radiation Protection ».*

L'unité de commande 60 est programmée pour piloter le scanner optique de balayage 30 de sorte à déplacer le motif le long d'un chemin de déplacement contenu dans le plan de focalisation.

### 3.2. Système optique de focalisation

Le système optique de focalisation 40 permet de focaliser le faisceau dans un plan de découpe 21.

Le système optique de focalisation 40 peut comporter :
- un orifice d'entrée 41 pour recevoir le faisceau dévié issu du scanner optique de balayage 30,
- un module concentrateur 46 pour focaliser le faisceau L.A.S.E.R. dans le plan de focalisation 21 et
- un orifice de sortie 43 pour envoyer le faisceau focalisé vers le tissu à traiter.

Le système optique de focalisation peut également comprendre un module de positionnement en profondeur pour déplacer le plan de focalisation.

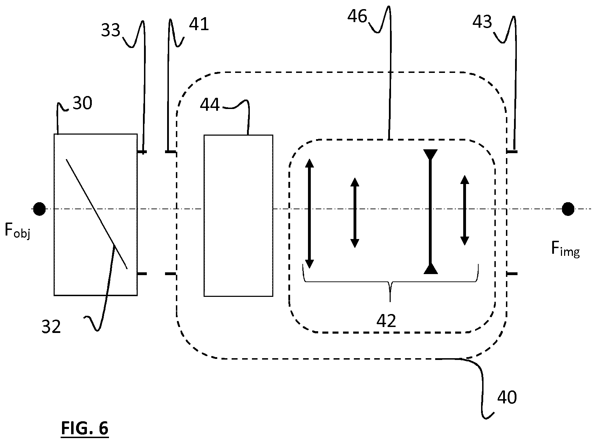

Par exemple en référence à la figure 6, le module concentrateur 46 comprend une pluralité de lentilles 42 (convergente et/ou divergente) et le module de positionnement en profondeur comprend un (ou plusieurs) moteur(s) (non représentés) associé(s) à une (ou plusieurs) lentille(s) 42 du module concentrateur 46 pour permettre son (leur) déplacement en translation le long du chemin optique du faisceau afin de faire varier la profondeur du plan de focalisation.

En variante, le système optique de focalisation peut être dépourvu de module de positionnement en profondeur. La modification de la position du plan de focalisation peut alors être obtenue en modifiant la divergence du faisceau en amont du système optique de focalisation, ou en déplaçant en profondeur l'appareil de découpe (ou le dispositif de conditionnement) devant le tissu cible à traiter.

Le module concentrateur 46 est défini optiquement par :
- un diaphragme d'ouverture (i.e. élément du module concentrateur 46 qui limite le plus la traversée du faisceau : il peut s'agir d'un diaphragme mécanique ou des limites d'une des lentilles du module concentrateur 46),
- une pupille d'entrée (i.e. image du diaphragme d'ouverture à travers la partie du module concentrateur 46 située en amont du diaphragme d'ouverture le long du chemin optique du faisceau),
- une pupille de sortie (i.e. image du diaphragme d'ouverture à travers la partie du module concentrateur 46 située en aval du diaphragme d'ouverture le long du chemin optique du faisceau).

La pupille d'entrée du module concentrateur 46 peut être positionnée dans le plan de la lentille équivalente 45 du système optique de focalisation 40. Ceci permet d'une part d'étaler l'énergie restante sur une plus grande surface de la rétine, et d'autre part de faciliter l'optimisation du dispositif de découpe selon l'invention.

Le système optique de focalisation 40 peut également comprendre un dispositif de relais optique 44 *(« relay lens »* en anglais) formant dispositif de transport de pupille d'entrée. Le dispositif de relais optique 44 permet d'imager d'une zone au voisinage du (ou des) miroir(s) optique(s) 32 du scanner optique de balayage 30 sur la pupille d'entrée du module concentrateur 46. Cette zone peut correspondre au milieu du (ou des) miroir(s) optique(s) 32 (i.e. le centre structurel du (ou des) miroir(s) optique(s) 32). Le dispositif de relais optique 44 se compose d'une pluralité de lentilles (par exemple convexes). Un tel dispositif de relais optique 44 est connu de l'homme du métier et ne sera pas décrit plus en détails dans la suite.

L'unité de commande 60 est programmée pour piloter le déplacement de la (ou des) lentille(s) du système optique de focalisation 40 le long d'un chemin optique du faisceau de sorte à le déplacer dans le plan de focalisation 21 en au moins trois plans de découpe respectifs de sorte à former un empilement de plans de découpe du tissu 20. Ceci permet d'effectuer une découpe dans un volume, par exemple dans le cadre d'une chirurgie réfractive.

Avantageusement, le module concentrateur 46 peut être déplaçable en translation le long du chemin optique du faisceau L.A.S.E.R. entre des première et deuxième positions extrêmes, le module concentrateur 46 étant plus proche du dispositif de relais optique 44 dans la première position extrême que dans la deuxième position extrême. Ceci permet de régler la profondeur du plan de focalisation correspondant au plan de découpe.

Le dispositif de découpe peut en outre comprendre un système optique pré-compensatoire - intégrant par exemple des lentilles dont certaines sont mobiles - positionné en amont du système optique de focalisation. Ce système optique pré-compensatoire permet de générer une aberration compensatoire en sortie du système optique de focalisation 40.

En effet, lorsque le faisceau L.A.S.E.R. issu du dispositif de découpe traverse :
- une interface de couplage (telle que décrite par exemple dans le document) positionnée entre le dispositif de découpe et l'œil du patient, et
- les tissus (cornée et cristallin) de l'œil du patient,
le faisceau L.A.S.E.R. subit des déformations induisant la formation d'aberrations.

L'intégration d'un système optique pré-compensatoire permet de corriger ces aberrations subies par le faisceau L.A.S.E.R. L'agencement du système optique pré-compensatoire - et en particulier les positions des lentilles mobiles du système optique pré-compensatoire - peut être déterminé par un calculateur en fonction de l'aberration compensatoire désirée en utilisant toute technique connue de l'homme du métier.

### 3.3. Système de mise en forme

Le système de mise en forme spatiale 50 du faisceau permet de faire varier le front d'onde du faisceau pour obtenir des points d'impact séparés les uns des autres dans le plan focal.

Plus précisément, le système de mise en forme 50 permet de moduler la phase du faisceau issu du laser femtoseconde 10 pour former des pics d'intensité dans le plan focal du faisceau, chaque pic d'intensité produisant un point d'impact respectif dans le plan focal correspondant au plan de découpe. Le système de mise en forme 50 est, selon le mode de réalisation illustré, un modulateur spatial de lumière à cristaux liquides, connu sous le sigle SLM, de l'acronyme anglais « *Spatial Light Modulator ».*

Le SLM permet de moduler la répartition finale d'énergie du faisceau, notamment dans le plan focal 21 correspondant au plan de découpe. Plus précisément, le SLM est adapté pour modifier le profil spatial du front d'onde du faisceau primaire issu du laser femtoseconde 10 pour distribuer l'énergie du faisceau L.A.S.E.R. en différents spots de focalisation dans le plan de focalisation.

La modulation en phase du front d'onde peut être vue comme un phénomène d'interférences en deux dimensions. Chaque portion du faisceau primaire issu du laser femtoseconde 10 est retardée ou avancée par rapport au front d'onde initial afin que chacune de ces portions soit redirigée de façon à réaliser une interférence constructive en N points distincts dans le plan focal d'une lentille. Cette redistribution d'énergie en une pluralité de points d'impact n'a lieu que dans un seul plan (i.e. le plan de focalisation) et pas tout au long du chemin de propagation du faisceau modulé. Ainsi, l'observation du faisceau modulé avant ou après le plan de focalisation ne permet pas d'identifier une redistribution de l'énergie en une pluralité de points d'impact distincts, du fait de ce phénomène qu'on peut assimiler à des interférences constructives (qui n'ont lieu que dans un plan et pas tout au long de la propagation comme dans le cas de la séparation d'un faisceau primaire en une pluralité de faisceaux secondaires).

Le SLM est un dispositif constitué d'une couche de cristaux liquides à orientation contrôlée permettant de façonner d'une manière dynamique le front d'onde, et donc la phase du faisceau. La couche de cristaux liquides d'un SLM est organisée comme une grille (ou matrice) de pixels. L'épaisseur optique de chaque pixel est contrôlée électriquement par orientation des molécules de cristal liquide appartenant à la surface correspondant au pixel. Le SLM exploite le principe d'anisotropie des cristaux liquides, c'est-à-dire la modification de l'indice des cristaux liquides, en fonction de leur orientation spatiale. L'orientation des cristaux liquides peut être réalisée à l'aide d'un champ électrique. Ainsi, la modification de l'indice des cristaux liquides modifie le front d'onde du faisceau L.A.S.E.R.

D'une manière connue, le SLM met en oeuvre un masque de phase, c'est-à-dire une carte déterminant comment la phase du faisceau doit être modifiée pour obtenir une répartition d'amplitude donnée dans son plan de focalisation. Le masque de phase est une image bidimensionnelle dont chaque point est associé à un pixel respectif du SLM. Ce masque de phase permet de piloter l'indice de chaque cristal liquide du SLM en convertissant la valeur associée à chaque point du masque - représentée en niveaux de gris compris entre 0 et 255 (donc du noir au blanc) - en une valeur de commande - représentée en une phase comprise entre 0 et 2π. Ainsi, le masque de phase est une consigne de modulation affichée sur le SLM pour entraîner en réflexion un déphasage spatial inégal du faisceau primaire illuminant le SLM. Bien entendu, l'homme du métier appréciera que la plage de niveau de gris peut varier en fonction du modèle de SLM utilisé. Par exemple dans certains cas, la plage de niveau de gris peut être comprise entre 0 et 220. Le masque de phase est généralement calculé par un algorithme itératif basé sur la transformée de Fourier, ou sur divers algorithmes d'optimisation, tels que des algorithmes génétiques, ou le recuit simulé. Différents masques de phase peuvent être appliqués aux SLM en fonction du nombre et de la position des points d'impact souhaités dans le plan focal du faisceau. Dans tous les cas, l'homme du métier sait calculer une valeur en chaque point du masque de phase pour distribuer l'énergie du faisceau en différents spots de focalisation dans le plan de focal.

Le SLM permet donc, à partir d'un faisceau L.A.S.E.R. gaussien générant un unique point d'impact, et au moyen du masque de phase, de répartir son énergie par modulation de phase de sorte à générer simultanément plusieurs points d'impact dans son plan de focalisation à partir d'un unique faisceau L.A.S.E.R. mis en forme par modulation de phase (un seul faisceau en amont et en aval du SLM).

En plus d'une diminution du temps de découpe de la cornée, la technique de modulation de la phase du faisceau L.A.S.E.R. permet d'autres améliorations, telles qu'une meilleure qualité de surface après découpe ou une diminution de la mortalité endothéliale. Les différents points d'impact du motif peuvent, par exemple, être régulièrement espacés sur les deux dimensions du plan focal du faisceau L.A.S.E.R., de manière à former un quadrillage de spots L.A.S.E.R.

Le recours à un système de relais de pupille induit la formation d'un foyer intermédiaire dans le trajet du faisceau. Pour les impulsions laser femtoseconde, ceci peut conduire à une intensité au foyer intermédiaire telle que des effets non-linéaires se produisent au point focal du type effet Kerr ou claquage et génération de plasma. Il en résulte alors une dégradation de la qualité optique du faisceau. Il convient donc de choisir une configuration optique dans laquelle la taille du faisceau au foyer intermédiaire permet de maintenir l'intensité suffisamment faible pour ne pas générer ces effets.

En ce sens, l'utilisation d'un dispositif de mise en forme de faisceau a pour effet de répartir l'énergie sur *« n* » points dans le plan de focalisation. L'intensité est donc divisée par « *n* » par rapport à un faisceau non mis en forme. La mise en forme du faisceau permet donc de véhiculer plus d'énergie dans un même faisceau sans générer d'effets non linéaires.

### 3.4. Unité de commande

Comme indiqué précédemment, l'unité de commande 60 permet de contrôler les différents éléments constituant l'appareil de découpe, à savoir le laser femtoseconde 10, le scanner optique de balayage 30 et le système optique de focalisation 40 et le système de mise en forme 50.

L'unité de commande 60 est connectée à ces différents éléments par l'intermédiaire d'un (ou plusieurs) bus de communication permettant :
- la transmission de signaux de commande tels que
   - le masque de phase au système de mise en forme,
   - le signal d'activation au laser femtoseconde,
   - la vitesse de balayage au scanner optique de balayage 30,
   - la position du scanner optique de balayage 30 le long du chemin de déplacement,
   - la profondeur de découpe au système optique de focalisation.
- la réception de données de mesure issues des différents éléments du système tels que
   - la vitesse de balayage atteinte par le scanner optique 30, ou
   - la position du système optique de focalisation, etc.

L'unité de commande 60 peut être composée d'une (ou plusieurs) station(s) de travail, et/ou d'un (ou plusieurs) ordinateur(s) ou peut être de tout autre type connu de l'homme du métier. L'unité de commande 60 peut par exemple comprendre un téléphone portable, une tablette électronique (tel qu'un IPAD^{®}), un assistant personnel (ou *« PDA* », sigle de l'expression anglo-saxonne « *Personal Digital Assistant »),* etc. Dans tous les cas, l'unité de commande 60 comprend un processeur programmé pour permettre le pilotage du laser femtoseconde 10, le scanner optique de balayage 30, le système optique de focalisation 40, le système de mise en forme 50, etc.

### 4. Théorie relative à l'invention et avantages vis-à-vis des dispositifs de l'art antérieur

L'appareil de découpe décrit précédemment permet de traiter une cataracte par focalisation d'un faisceau laser dans différentes zones du cristallin de manière à le fragmenter en morceaux de taille suffisamment petite pour être aspiré par une sonde d'irrigation-aspiration.

La focalisation du faisceau issu du laser femtoseconde se fait au moyen d'un dispositif de traitement de faisceau situé entre le laser femtoseconde et l'œil du patient.

Le faisceau issu du laser femtoseconde est composé d'une succession d'impulsions temporelles. D'une impulsion à l'autre, le point focal est déplacé dans le cristallin de manière à générer une succession d'impacts. Le déplacement du point focal est réalisé par déviation du faisceau au moyen du système optique de déviation.

Pour chaque impulsion, une partie de l'énergie de l'impulsion est consommée par la vaporisation du tissu. Le reste de l'énergie est absorbée par la rétine, ce qui provoque localement son échauffement.

Un grand nombre d'impulsions sont nécessaires pour découper le cristallin. L'énergie totale absorbée par la rétine et l'échauffement qui en résulte représente une limite pour la technologie de traitement par laser femtoseconde.

Un dispositif de traitement de faisceau de l'art antérieur est représenté sur la figure 1. Ce dispositif de traitement comprend un scanner de balayage 1 et un ensemble de focalisation 2. Les miroirs du scanner de balayage sont placés de part et d'autre, et à équidistance d'une première position correspondant à la pupille d'entrée de l'ensemble de focalisation 2.

Pour un miroir en position 0, le rayon 9b est parallèle à l'axe optique de l'ensemble de focalisation 2. Il est focalisé par l'ensemble de focalisation 2 dans le plan focal image 3 situé dans le cristallin. Une partie de l'énergie du faisceau focalisé génère un dommage au point 3b. L'énergie restante continue sa propagation le long de l'axe optique et rencontre la rétine autour du point 4b. La taille du faisceau est alors agrandie du fait de la divergence du faisceau résultante de la focalisation.

Pour un miroir en position angulaire maximum, le rayon est dévié de l'axe optique suivant le chemin 9a. Il est focalisé par l'ensemble de focalisation 2 au point 3a du cristallin, à une distance h du centre. L'énergie restante rencontre la rétine autour du point 4a situé à la distance h' du centre de la rétine.

De même, pour un miroir en position angulaire minimum, le rayon est dévié de l'axe optique suivant le chemin 9c. Il est focalisé par l'ensemble de focalisation 2 au point 3c du cristallin. L'énergie restante rencontre la rétine autour du point 4c.

Dans les dispositifs de traitement de faisceau actuellement utilisés pour la chirurgie de la cataracte, l'angle effectué par les rayons en sortie du dispositif de traitement est tel que le faisceau tend à se rapprocher de l'axe optique avec la propagation. Ceci est dû à la position des miroirs de scanner qui sont situés avant le foyer objet de l'ensemble de focalisation 2. Ainsi, la distance h' est inférieure à la distance h : la surface sur laquelle est répartie l'énergie sur la rétine est donc plus faible que la surface balayée par le cristallin. L'énergie totale que l'on peut envoyer dans le cristallin est limitée par ce faible étalement.

Dans le cadre de la présente invention, les miroirs du scanner optique de balayage 32 sont positionnés sur le chemin optique après le point focal objet F_{Objet} du système optique de focalisation 40. Dans cette configuration, le faisceau s'éloigne de l'axe optique à la sortie du système optique de focalisation 40. On obtient alors un étalement 23 plus important de l'énergie sur la rétine 22, ce qui conduit à une élévation de température plus faible. Ceci a pour effet de repousser les limites d'énergie totale cumulée utilisée lors de la chirurgie.

Cet effet d'étalement est d'autant plus important que la position du scanner est éloignée du foyer objet F_{Objet}. La pupille d'entrée du système optique de focalisation 40 peut par exemple être placée dans le plan de la lentille équivalente 45 du système optique de focalisation 40.

Une telle configuration peut être par exemple réalisée en utilisant un dispositif de relais de pupille placé de telle manière à imager le milieu des miroirs du scanner dans le plan de la lentille équivalente 45 au système optique de focalisation 40.

### 5. Conclusions

Ainsi, l'invention permet de disposer d'un outil de découpe efficace.

Le positionnement du (ou des) miroir(s) optique(s) pivotant du scanner optique de balayage 30 entre le plan focal objet F_{Objet} du système optique de focalisation 40 et le système de focalisation 40 permet un étalement plus important de l'énergie sur la rétine, et donc une élévation de température plus faible.

Il est ainsi possible d'augmenter la puissance du laser femtoseconde 10 sans dépasser les éclairements énergétiques limites susceptibles de dégrader la rétine.

L'invention a été décrite pour des opérations de découpes d'une cornée dans le domaine de la chirurgie ophtalmologique, mais il est évident qu'elle peut être utilisée pour d'autre type d'opération en chirurgie ophtalmologique sans sortir du cadre de l'invention. Par exemple, l'invention trouve une application dans la chirurgie réfractive cornéenne, tel que le traitement des amétropies, notamment myopie, hypermétropie, astigmatisme, dans le traitement de la perte d'accommodation, notamment la presbytie. L'invention trouve également une application dans le traitement de la cataracte avec incision de la cornée, découpe de la capsule antérieure du cristallin, et fragmentation du cristallin. Enfin, d'une manière plus générale, l'invention concerne toutes les applications cliniques ou expérimentales sur la cornée ou le cristallin d'un oeil humain ou animal. D'une manière encore plus générale, l'invention concerne le domaine large de la chirurgie au L.A.S.E.R. et trouve une application avantageuse lorsqu'il s'agit de vaporiser des tissus mous humains ou animaux, à teneur en eau élevée.

L'invention est définie dans les revendications suivantes :

## Revendications

1. Appareil de traitement d'un tissu humain ou animal, tel qu'une cornée ou un cristallin, ledit appareil incluant un dispositif de conditionnement d'un faisceau L.A.S.E.R. généré par un laser femtoseconde (10), le dispositif de conditionnement étant disposé en aval du laser femtoseconde et comprenant un scanner optique de balayage (30) et un système optique de focalisation (40) en aval du scanner optique de balayage (30) :
- Le scanner optique de balayage (30) comportant :
o au moins un miroir optique (32) pivotant autour d'au moins un axe pour dévier le faisceau L.A.S.E.R.,
- Le système optique de focalisation (40) comportant :
o un module concentrateur (46) pour focaliser le faisceau L.A.S.E.R. dans un plan de focalisation (21),
***caractérisé en* ce *que*** ledit au moins un miroir optique (32) pivotant du scanner optique de balayage (30) s'étend sur le chemin optique du faisceau L.A.S.E.R. entre :
- le plan focal objet F_{Objet} d'une lentille équivalente (45) au système optique de focalisation (40), et
- un orifice d'entrée (41) du système optique de focalisation (40).

2. Appareil de traitement selon la revendication 1, ***dans lequel*** une pupille d'entrée du module concentrateur (46) est positionnée dans le plan de la lentille équivalente (45) au système optique de focalisation (40).

3. Appareil de traitement selon l'une quelconque des revendications 1 ou 2, ***dans lequel*** le système optique de focalisation (40) comporte un dispositif de relais optique (44) en amont du module concentrateur (46).

4. Appareil de traitement selon la revendication précédente, ***dans lequel*** le dispositif de relais optique (44) est positionné le long du chemin optique du faisceau L.A.S.E.R. de sorte à former une image d'une zone au voisinage dudit au moins un miroir pivotant (32) dans le plan de la lentille équivalente au système optique de focalisation (40).

5. Appareil de traitement selon l'une quelconque des revendications 1 à 4, ***dans lequel*** le dispositif de conditionnement comprend en outre un système de mise en forme (50) du faisceau, pour moduler la phase du front d'onde du faisceau L.A.S.E.R. de sorte à obtenir un faisceau L.A.S.E.R. modulé en phase selon une consigne de modulation calculée pour répartir l'énergie du faisceau L.A.S.E.R. en au moins deux points d'impact formant un motif dans le plan de focalisation (21).

6. Appareil de découpe selon la revendication 1 à 5, ***dans lequel*** le dispositif de conditionnement comprend en outre une unité de commande (60) pour piloter le laser femtoseconde (10), le scanner optique de balayage (30) et le système optique de focalisation (40).

7. Appareil de découpe selon la revendication 3, ***dans lequel*** le module concentrateur (46) est déplaçable en translation le long du chemin optique du faisceau L.A.S.E.R. entre des première et deuxième positions extrêmes, le module concentrateur (46) étant plus proche du dispositif de relais optique (44) dans la première position extrême que dans la deuxième position extrême.

8. Appareil de découpe selon la revendication 7, ***lequel*** comprend en outre un dispositif optique pré-compensatoire positionné en amont du système optique de focalisation de sorte à générer une aberration compensatoire en sortie du système optique de focalisation, ladite aberration compensatoire permettant de compenser les aberrations produites sur le faisceau L.A.S.E.R. notamment par la traversée du tissu humain ou animal.

## Patentansprüche

1. Gerät zur Behandlung eines menschlichen oder tierischen Gewebes, wie beispielsweise einer Hornhaut oder einer Augenlinse, wobei das Gerät eine Vorrichtung zur Konditionierung eines LASER-Strahls umfasst, der durch einen Femtosekundenlaser (10) erzeugt wird, wobei die Konditionierungsvorrichtung dem Femtosekundenlaser nachgelagert angeordnet ist und einen optischen Abtastscanner (30) und ein optisches Fokussierungssystem (40) dem optischen Abtastscanner (30) nachgelagert umfasst:
- wobei der optische Abtastscanner (30) Folgendes umfasst:
o mindestens einen optischen Spiegel (32), der um mindestens eine Achse schwenkt, um den LASER-Strahl abzulenken,
- wobei das optische Fokussierungssystem (40) Folgendes umfasst:
o ein Konzentratormodul (46) zum Fokussieren des LASER-Strahls in einer Fokussierungsebene (21),
**dadurch gekennzeichnet, dass** der mindestens eine schwenkende optische Spiegel (32) des optischen Abtastscanners (30) sich auf dem Lichtweg des LASER-Strahls erstreckt zwischen:
- der Objektbrennebene F_{Objet} einer Linse (45), die dem optischen Fokussierungssystem (40) gleichwertig ist, und
- einer Eintrittsöffnung (41) des optischen Fokussierungssystems (40).

2. Behandlungsgerät nach Anspruch 1, wobei eine Eintrittspupille des Konzentratormoduls (46) in der Ebene der Linse (45) positioniert ist, die dem optischen Fokussierungssystem (40) gleichwertig ist.

3. Behandlungsgerät nach einem der Ansprüche 1 oder 2, wobei das optische Fokussierungssystem (40 eine optische Relaisvorrichtung (44) dem Konzentratormodul (46) vorgelagert umfasst.

4. Behandlungsgerät nach dem vorhergehenden Anspruch, wobei die optische Relaisvorrichtung (44) derart entlang des optischen Wegs des LASER-Strahls positioniert ist, dass ein Bild einer Zone in der Nachbarschaft des mindestens einen schwenkbaren Spiegels (32) in der Ebene der Linse gebildet wird, die dem optischen Fokussierungssystem (40) gleichwertig ist.

5. Behandlungsgerät nach einem der Ansprüche 1 bis 4, wobei die Konditionierungsvorrichtung ferner ein System zur Formgebung (50) des Strahls zum Modulieren der Phase der Wellenfront des LASER-Strahls derart umfasst, das ein modulierter LASER-Strahl erhalten wird, der gemäß einem Modulationseinstellwert in Phase moduliert wird, der berechnet wird, um die Energie des LASER-Strahls an mindestens zwei Auftreffpunkten zu verteilen, die ein Motiv in der Fokussierungsebene (21) bilden.

6. Schneidgerät nach Anspruch 1 bis 5, wobei die Konditionierungsvorrichtung ferner eine Steuereinheit (60) zum Lenken des Femtosekundenlasers (10), des optischen Abtastscanners (30) und des optischen Fokussierungssystems (40) umfasst.

7. Schneidgerät nach Anspruch 3, wobei das Konzentratormodul (46) translatorisch entlang des Lichtwegs des LASER-Strahls zwischen einer ersten und einer zweiten äußersten Position beweglich ist, wobei das Konzentratormodul (46) in der ersten äußersten Position näher an der optischen Relaisvorrichtung (44) ist als in der zweiten äußersten Position.

8. Schneidgerät nach Anspruch 7, das ferner eine optische Vorkompensationsvorrichtung umfasst, die dem optischen Fokussierungssystem derart vorgelagert positioniert ist, dass eine Kompensationsaberration am Ausgang des optischen Fokussierungssystems erzeugt wird, wobei die Kompensationsaberration das Kompensieren der, insbesondere durch die Durchquerung des menschlichen oder tierischen Gewebes, auf dem LASER-Strahl erzeugten Aberrationen ermöglicht.

## Claims

1. An apparatus for treating a human or animal tissue, such as a cornea or a crystalline lens, said apparatus including a device for conditioning a L.A.S.E.R. beam generated by a femtosecond laser (10), the conditioning device being disposed downstream of the femtosecond laser and comprising a sweeping optical scanner (30) and a focusing optical system (40) downstream of the sweeping optical scanner (30):
- The sweeping optical scanner (30) including:
o at least one optical mirror (32) pivoting around at least one axis for deflecting the L.A.S.E.R. beam,
- The focusing optical system (40) including:
o a concentrator module (46) for focusing the L.A.S.E.R. beam in a focusing plane (21),
**characterized in that** said at least one pivoting optical mirror (32) of the sweeping optical scanner (30) extends over the optical path of the L.A.S.E.R beam between:
- the object focal plane F_{object} of a lens (45) equivalent to the focusing optical system (40), and
- an inlet orifice (41) of the focusing optical system (40).

2. The treatment apparatus according to claim 1, wherein an entrance pupil of the concentrator module (46) is positioned in the plane of the lens (45) equivalent to the focusing optical system (40).

3. The treatment apparatus according to any one of claims 1 or 2, wherein the focusing optical system (40) includes an optical relay device (44) upstream of the concentrator module (46) .

4. The treatment apparatus according to the preceding claim, wherein the optical relay device (44) is positioned along the optical path of the L.A.S.E.R. beam so as to form an image of an area in the vicinity of said at least one pivoting mirror (32) in the plane of the lens equivalent to the focusing optical system (40).

5. The treatment apparatus according to any one of claims 1 to 4, wherein the conditioning device further comprises a beam shaping system (50) for modulating the phase of the wave front of the L.A.S.E.R. beam so as to obtain a phase-modulated L.A.S.E.R. beam according to a modulation set value calculated to distribute the energy of the L.A.S.E.R. beam into at least two impact points forming a pattern in the focusing plane (21).

6. A cutting apparatus according to claims 1 to 5, wherein the conditioning device further comprises a control unit (60) for driving the femtosecond laser (10), the sweeping optical scanner (30) and the focusing optical system (40).

7. The cutting apparatus according to claim 3, wherein the concentrator module (46) is movable in translation along the optical path of the L.A.S.E.R. beam between first and second extreme positions, the concentrator module (46) being closer to the optical relay device (44) in the first extreme position than in the second extreme position.

8. The cutting apparatus according to claim 7, which further comprises a pre-compensatory optical device positioned upstream of the focusing optical system so as to generate a compensatory aberration at the outlet of the focusing optical system, said compensatory aberration making it possible to compensate for the aberrations produced on the L.A.S.E.R. beam in particular by passing through the human or animal tissue.
